# FASCICULE DE BREVET EUROPEEN

(11) **EP 2 650 583 B1**
(45) Date de publication et mention de la délivrance du brevet: **13.05.2015**
(21) Numéro de dépôt: 13160389.6
(22) Date de dépôt: 21.03.2013
(51) Int. Cl.: F16M 11/10, F16M 11/24, F16M 11/20

(54) **Bras porteur de charge réglable, dispositif de suspension d'une charge**
Regulierbarer Lastenträgerarm, und Aufhängevorrichtung für eine Last
Adjustable load-bearing arm, device for suspending a load

(30) Priorité: 10.04.2012 FR 1253259
(43) Date de publication de la demande: 16.10.2013
(73) Titulaire: Maquet SAS, 45160 Ardon (FR)
(72) Inventeur: Senelier, Grégory, 41210 LA MAROLLE EN SOLOGNE (FR); Theodon, Benoit, 45380 La Chapelle St Mesmin (FR)
(74) Mandataire: Prugneau, Philippe

(56) Documents cités:
- DE-A1- 1 466 999
- DE-A1- 19 501 028
- FR-A1- 2 908 497
- US-A- 3 160 379
- US-A1- 2004 084 587
- US-A1- 2004 188 578
- US-A1- 2011 315 843

## Description

### Domaine technique

L'invention concerne un bras porteur de charge à position réglable comprenant une ossature globalement en forme de parallélogramme déformable pourvue d'au moins une biellette fixe, une biellette mobile, une première et une seconde bielles mobiles et une extrémité libre apte à supporter une charge.

L'invention concerne aussi un dispositif de suspension de charge comprenant un tel bras porteur.

### Technique antérieure

Plus particulièrement, l'invention s'applique à un bras porteur pour dispositif de suspension destiné à être utilisé en milieu médical, en particulier en salle d'opération.

Un dispositif de suspension pour salle d'opération comprend de manière générale un ou plusieurs bras porteurs, pouvant être articulés et suspendus au plafond de la salle d'opération, destinés à supporter une charge comme par exemple un équipement tel qu'un dispositif d'éclairage, un écran, une caméra, etc., ou un dispositif médical tel qu'un flacon de transfusion ou de perfusion, etc.

De manière connue, le bras porteur comporte au moins un élément basculant en forme de parallélogramme déformable, grâce auquel le bras porteur permet à l'utilisateur de modifier facilement et de façon stable la position angulaire du bras pour positionner manuellement la charge par exemple à une hauteur souhaitée. L'utilisateur peut alors installer ou enlever une charge après avoir bloquer le bras porteur en position.

Pour ce faire, il est connu d'équiper le bras porteur tel que décrit plus haut d'un mécanisme comprenant un vérin pneumatique pouvant être accompagné d'un mécanisme de blocage du vérin. On connait par exemple un tel bras porteur du document de brevet FR 2 899 309. Un inconvénient d'un tel bras porteur est que le vérin est adapté pour supporter une gamme de poids de charges réduites. En outre, le vérin de ce bras porteur est fixé de telle sorte à permettre d'une part de positionner le bras porteur dans une position souhaitée quelconque lorsque le bras porteur porte une charge et d'autre part d'entraîner le bras porteur dans une position de repos prédéterminée lorsque le bras porteur est à vide. Pour installer une charge à l'extrémité libre de l'élément basculant, l'utilisateur doit positionner le bras porteur dans cette position de blocage prédéterminée dans laquelle le mécanisme de blocage du vérin bloque le bras porteur. L'utilisateur doit ensuite déplacer le bras porteur au-delà de sa position de blocage pour pouvoir le débloquer et le positionner où il le souhaite. Cette position de blocage prédéterminée unique peut dans certains cas être difficile d'accès pour l'utilisateur et non adaptée à l'installation d'une charge.

On connaît encore des documents de brevet US-2004/188578, US-2011/315843, FR-2908497, US-2004/084587 et DE-19501028, un bras porteur de charge à position réglable.

### Exposé de l'invention

Le but de l'invention est de proposer un autre bras porteur de charge à position réglable apte à supporter des charges de poids très variés et pouvant être chargé dans plusieurs positions.

A cet effet, l'invention a pour objet un bras porteur de charge à position réglable comprenant une ossature globalement en forme de parallélogramme déformable pourvue d'au moins une biellette fixe, une biellette mobile, une première et une seconde bielles mobiles et une extrémité libre apte à supporter une charge, un vérin dont le corps et le piston sont l'un couplé à la biellette fixe, l'autre à au moins l'une des première, seconde bielles, ledit vérin étant apte à actionner lesdites première, et seconde bielles par rapport à ladite biellette fixe de sorte à déformer ledit parallélogramme déformable, ledit vérin étant de type à pression réglable de telle sorte que la pression à l'intérieur dudit vérin soit réglable en fonction du poids de ladite charge portée par ledit bras porteur, caractérisé en ce qu'il comporte, en ladite extrémité libre, une pièce de connexion apte à porter ladite charge et à être solidaire :
- dans une première configuration, de ladite biellette mobile de sorte à présenter un axe d'orientation fixe par rapport à ladite biellette fixe et variable par rapport auxdites première et seconde bielles lorsque ledit bras porteur change de position,
- dans une seconde configuration, de l'une au moins desdites première et seconde bielles de sorte à présenter un axe d'orientation variable par rapport à ladite biellette fixe et fixe par rapport auxdites première et seconde bielles lorsque ledit bras porteur change de position.

Avec un tel bras porteur de charge, lorsqu'un utilisateur installe une charge sur le bras porteur, par exemple pour remplacer une charge par une autre charge d'un poids différent, plus lourde ou plus légère, il règle la pression intérieure du vérin en fonction du poids de cette autre charge. Le bras porteur selon l'invention est donc compatible avec une grande variété d'équipements ou de dispositifs. En plus, l'utilisateur peut fixer sur le bras porteur une charge dont l'axe restera soit parallèle à la largeur du parallélogramme, c'est-à-dire en conservant la verticalité de la charge quelle que soit l'inclinaison du bras porteur, ou soit à la longueur du parallélogramme, c'est-à-dire que l'axe de la charge suit l'inclinaison du vérin du bras porteur quelle que soit l'inclinaison du bras porteur ;

Un tel bras porteur de charge selon l'invention peut présenter avantageusement les particularités suivantes :
- le bras porteur comprend un dispositif de blocage de la position angulaire de la première, seconde bielle par rapport à la biellette fixe, le dispositif de blocage comprenant une crémaillère et un loquet solidaires l'un du corps du vérin l'autre du piston du vérin, le loquet étant agencé pour être et mobile le long de la crémaillère et apte à coopérer avec ladite crémaillère de sorte à bloquer la position angulaire de la première, seconde bielle par rapport à la biellette fixe selon une pluralité de positions de blocage. Avantageusement, le changement de charge est donc facile et ergonomique pour l'utilisateur qui peut bloquer le bras porteur dans une position souhaitée quelconque afin d'installer une charge sur le bras porteur ou d'effectuer un changement de charge;
- le loquet comprend au moins un cran destiné à engager une encoche de la crémaillère pour bloquer le bras porteur en position. L'engagement du cran dans une encoche de la crémaillère permet un blocage très efficace du loquet sur la crémaillère et par là du bras porteur, pour une large gamme de poids de charge. A titre de comparaison, des dispositifs de blocage par friction ne suffisent pas pour retenir le bras porteur si la charge est trop lourde ;
- le bras porteur comprend une valve couplée au vérin et destinée à relier ledit vérin à un dispositif de réglage de la pression du vérin ;
- le bras porteur comprend au moins une coque formant la seconde bielle enveloppant la première bielle;
- la première bielle présente une forme de Y dont la base est montée pivotante sur la biellette fixe, dont l'extrémité libre d'une branche est montée pivotante sur la biellette mobile et l'extrémité libre de l'autre branche est reliée à une extrémité du vérin, l'autre extrémité du vérin étant pivotante par rapport à la biellette fixe..

L'invention s'étend à un dispositif de suspension d'une charge comprenant au moins un bras articulé ayant un premier bras agencé pour être mobile en rotation selon un mouvement de rotation dans un premier plan et un bras porteur selon l'invention relié au premier bras par une articulation pivot agencée pour autoriser sa rotation selon un mouvement de rotation dans un deuxième plan distinct du premier plan.

### Description sommaire des dessins

La présente invention sera mieux comprise et d'autres avantages apparaîtront à la lecture de la description détaillée d'un mode de réalisation pris à titre d'exemple nullement limitatif et illustré par les dessins annexés dans lesquels :
- la figure 1 est une vue en perspective d'un dispositif de suspension d'une charge comprenant un bras porteur selon l'invention ;
- la figure 2 est une vue longitudinale du bras porteur de la figure 1, décapoté à l'avant, dans une première position;
- la figure 3 est une vue en perspective arrière du bras porteur de la figure 2 ;
- la figure 4 est un agrandissement en perspective d'un détail du bras porteur de la figure 3 ;
- la figure 5 est une vue en perspective du bras porteur de la figure 2 dans sa première position ;
- la figure 6 est un agrandissement en perspective d'un détail du bras porteur de la figure 5 ;
- la figure 7 est une vue selon la coupe VII- VII du bras porteur de la figure 6;
- la figure 8 est une vue en perspective du bras porteur de la figure 2 dans une deuxième position ;
- la figure 9 est un agrandissement en perspective d'un détail du bras porteur de la figure 8;
- la figure 10 est une vue longitudinale du bras porteur de la figure 2, décapoté à l'avant, dans sa deuxième position ;
- la figure 11 est une vue longitudinale similaire à la figure 10 du bras porteur dans une autre position.

### Description d'un mode de réalisation

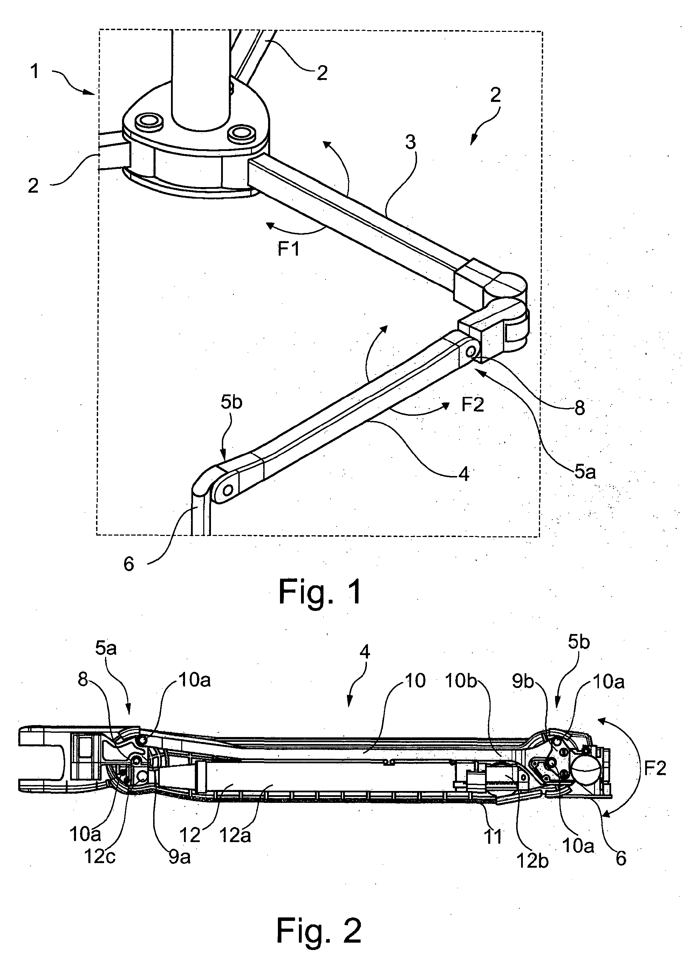

Sur la figure 1, on a représenté schématiquement un dispositif de suspension 1 d'une charge (non représentée) pour salle d'opération, conçu ici pour être suspendu au plafond de la salle d'opération, et comprenant dans l'exemple illustré trois bras articulés 2 dont un seul est montré dans son ensemble.

Chaque bras articulé 2 comprend un premier bras 3 dit d'extension autorisant un mouvement de rotation du bras articulé 2 selon la double flèche F1 dans un premier plan, ici sensiblement parallèle au plafond de la salle d'opération c'est-à-dire horizontal. Le premier bras 3 est relié par une articulation pivot à un second bras dit de compensation ou bras porteur 4 autorisant un mouvement de rotation du bras articulé 2 autour d'un axe de pivot 8 selon la double flèche F2 dans un deuxième plan sensiblement perpendiculaire au premier plan, c'est-à-dire ici vertical.

On comprendra que le bras porteur 4 selon l'invention pourrait sans sortir du cadre de l'invention être monté sur pivot de toute autre manière, par exemple sur un autre type de dispositif de suspension ou directement sur un mur ou un plafond d'une salle.

L'extrémité libre du bras porteur 4 est destinée à supporter, via une pièce de connexion 6, une charge (non représentée) qui peut être par exemple un équipement, tel qu'un dispositif d'éclairage, écran, caméra, etc., ou un dispositif médical, tel qu'un flacon de transfusion ou de perfusion, etc.

Comme on peut le voir sur la figure 2, le bras porteur 4 comprend, une coque 11 dont les extrémités 5a, 5b définissent les extrémités du bras porteur 4. Enveloppé dans cette coque 11, le bras porteur 4 comprend également une biellette fixe 9a solidaire d'une extrémité 5a de la coque 11 et une biellette mobile 9b montée pivotante en l'extrémité dite libre 5b de la coque 11. La biellette fixe 9a et la biellette mobile 9b sont reliées entre elles par une bielle 10 montée pivotante en ses deux extrémités par rapport aux biellettes fixe 9a et mobile 9b. Ainsi, la coque 11, la bielle 10, la biellette fixe 9a et la biellette mobile 9b forment globalement un parallélogramme déformable à quatre points.

Le bras porteur 4 comprend également visible sur la figure 2 un vérin 12, par exemple pneumatique, reliant la biellette fixe 9a à la bielle 10 et à la coque 11. Selon un autre mode de réalisation, le vérin peut être relié à l'une seulement de la bielle et de la coque ou à la biellette mobile. Le vérin 12 permet ainsi d'actionner le parallélogramme déformable pour régler la position angulaire ou inclinaison du bras porteur 4 par rapport à la biellette fixe 9a en faisant pivoter le bras porteur 4 selon la double-flèche F2 autour de l'axe de pivot 8. Le parallélogramme déformable et le vérin 12 permettent ainsi à l'utilisateur de modifier facilement et de façon stable la position du bras porteur 4 pour positionner manuellement la charge à une hauteur souhaitée.

Le vérin 12 est constitué, de manière connue en soi, par un corps 12a dans lequel coulisse un piston 12b. Le corps 12a est monté à son extrémité libre pivotant sur la biellette fixe 9a selon un axe 12c décalé par rapport à l'axe de pivot 8, et l'extrémité libre du piston 12b est reliée à la bielle 10 et à la coque 11 comme décrit ci-dessous.

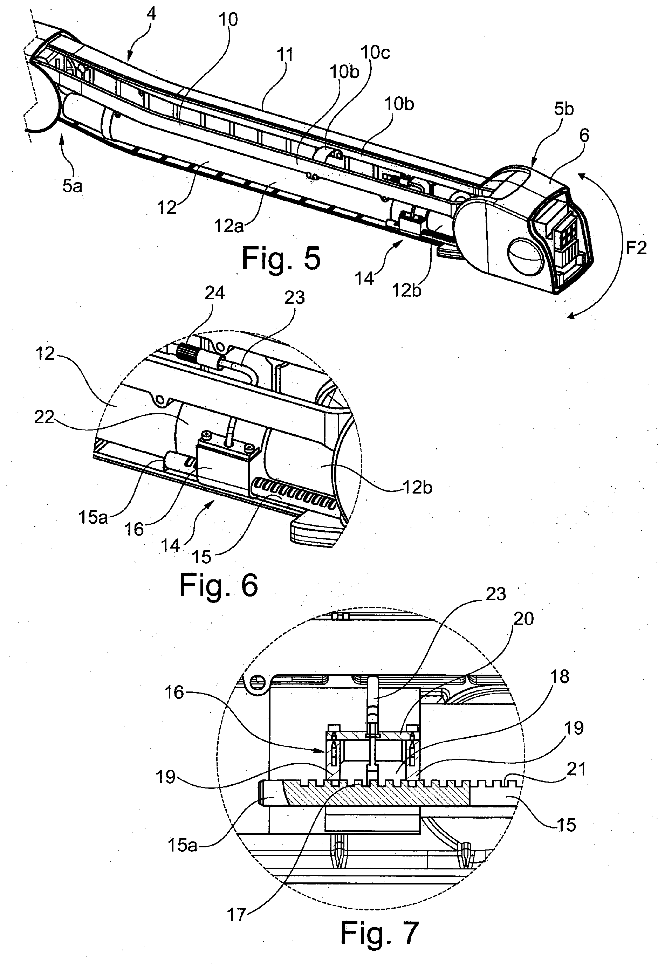

La bielle 10 comprend ici avantageusement deux tiges 10b sensiblement identiques, parallèles l'une à l'autre de sorte à encadrer le vérin 12, et pouvant être reliées par une ou plusieurs traverses 10c, comme on peut mieux le voir sur la figure 5. La bielle 10 a ici une forme de Y, la base du Y étant montée pivotante sur la biellette fixe 9a selon un point de pivot 10a de la coque 11 décalé par rapport à l'articulation pivot 8 du bras porteur 4. L'extrémité libre d'une branche du Y est montée pivotante sur la biellette mobile 9b par rapport à un point pivot 10a, biellette mobile 9b étant elle-même pivotante par rapport à un autre point pivot 10a prévu au niveau de l'extrémité libre 5b du bras porteur 4. L'extrémité libre de l'autre branche du Y est reliée à l'extrémité libre du piston 12b du vérin 12.

Avantageusement, le vérin 12 est un vérin pneumatique à pression réglable de telle sorte que la pression à l'intérieur du vérin 12 peut être réglée en fonction du poids de la charge portée par le bras porteur 4, comme cela sera décrit plus en détail ci-dessous. Le gaz du vérin 12 peut être par exemple de l'air ou de l'azote. La pression maximale à l'intérieur du vérin 12 est avantageusement choisie en fonction du poids de la charge la plus lourde susceptible d'être installée sur le bras porteur 4. Par exemple, en réglant la pression à l'intérieur du vérin entre 12 à 30 bars, le bras porteur 4 peut supporter une charge allant jusqu'à 40 kg. De préférence, le vérin 12 est agencé pour disposer d'une large gamme de poids comprise entre 5 kg et 40 kg.

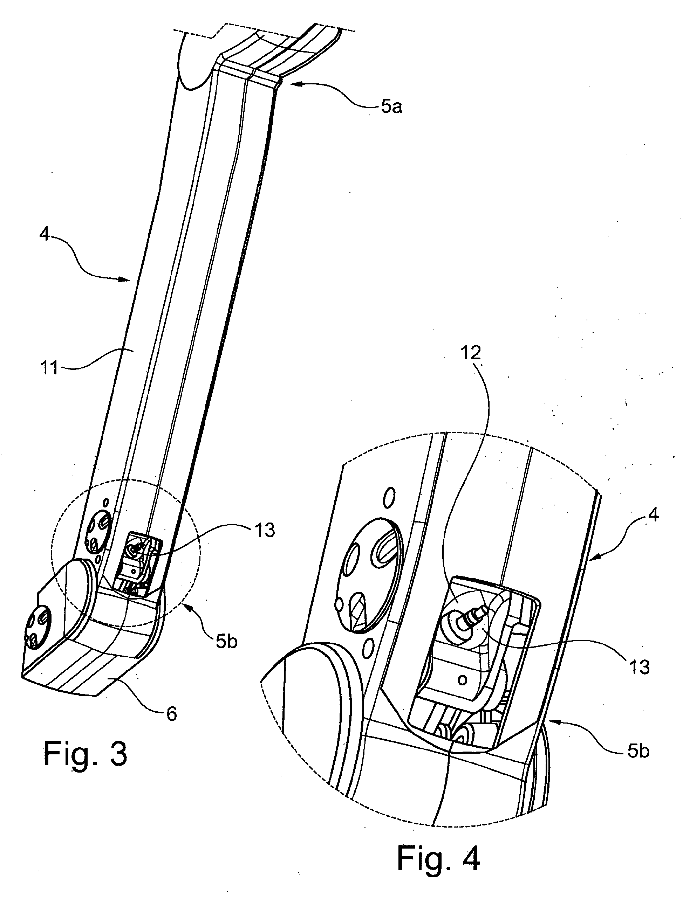

Comme on peut le voir sur les figures 3 et 4, le bras porteur 4 comprend une valve 13 ici disposée directement sur le vérin 12 pour le relier à un dispositif de réglage de la pression (non représenté) tel qu'un compresseur ou un réservoir sous pression par exemple d'air ou d'azote. Le dispositif de réglage de la pression peut être connecté à la valve 13 par un embout de gonflage à enfoncer (non représenté). Pour déconnecter le dispositif de réglage de la pression, on pourra avantageusement prévoir, adjacent à la valve 13, un bouton (non représenté) permettant de libérer l'embout de la valve 13. La valve 13 pourrait sans sortir du cadre de l'invention être disposée sur la coque 11, de préférence sur une face arrière de la coque 11, et reliée au vérin 12 par un raccord flexible. On comprendra que pour des raisons d'ergonomie, la valve 13 est de préférence disposée le long du bras porteur 4 du côté de l'extrémité libre 5 du bras porteur 4 de sorte à être au plus bas et faciliter l'accès à l'utilisateur pour effectuer facilement un réglage de la pression dans le vérin 12.

Comme visible sur la figure 5, le bras porteur 4 comporte en outre un dispositif de blocage 14 permettant de bloquer le bras porteur 4 selon une pluralité de positions de blocage angulaires, ou inclinaison, du bras porteur 4 selon la double-flèche F2. Le dispositif de blocage 14 est de préférence de type mécanique, et peut être composé par exemple de deux pièces mécaniques complémentaires, par exemple munies respectivement de dents ou de crans coopérant avec des encoches, fixées respectivement au corps 12a et au piston 12b du vérin 12. Dans l'exemple représenté sur la figure 6, le dispositif de blocage 14 comprend une crémaillère 15 fixée à l'extrémité libre du piston 12b et coopérant avec un loquet 16 solidaire du corps 12a du vérin 12 et mobile le long de la crémaillère 15. Le loquet 16 comprend ici un manchon 22 entourant plus précisément le corps 12a du vérin 12 pour solidariser le loquet 16 et le vérin 12.

Plus particulièrement, comme on peut mieux le voir sur la figure 7, le loquet 16 comprend au moins un cran 17, ici une pluralité de crans 17, destiné à s'engager dans des encoches 21 de la crémaillère 15 pour bloquer le bras porteur 4 dans une position de blocage angulaire choisie. On comprendra que les crans 17 et les encoches 21 sont configurés de sorte à avoir un même pas et un même module. Dans l'exemple représenté sur la figure 7, les crans 17 du loquet 16 sont formés sur un élément 18 mobile entre deux montants 19 d'une potence 20 de sorte à pouvoir occuper une position basse (représentée sur la figure 7) dans laquelle les crans 17 sont engagés dans les encoches 21 de la crémaillère 15 pour bloquer tout mouvement du corps 12a du vérin 12 par rapport au piston 12b du vérin 12 et ainsi bloquer le bras porteur 4 dans la position angulaire qu'il occupe, et une position haute du loquet 16 (non représentée) dans laquelle les crans 17 sont désengagés des encoches 21 de la crémaillère 15 pour permettre un mouvement du corps 12a du vérin 12 par rapport au piston 12b du vérin 12 et donc le déplacement angulaire du bras porteur 4 selon la double-flèche F2 représentée par exemple figure 5.

L'élément 18 mobile du loquet 16 est par ailleurs relié à un raccord flexible 23 courant le long du vérin 12 et ayant à son extrémité libre opposée à l'élément 18 une molette 24 permettant à un utilisateur de faire coulisser l'élément 18 entre les montants 19. La molette 24 permet en outre à l'utilisateur de bloquer facilement l'élément 18 en position haute ou basse sur la crémaillère 15.

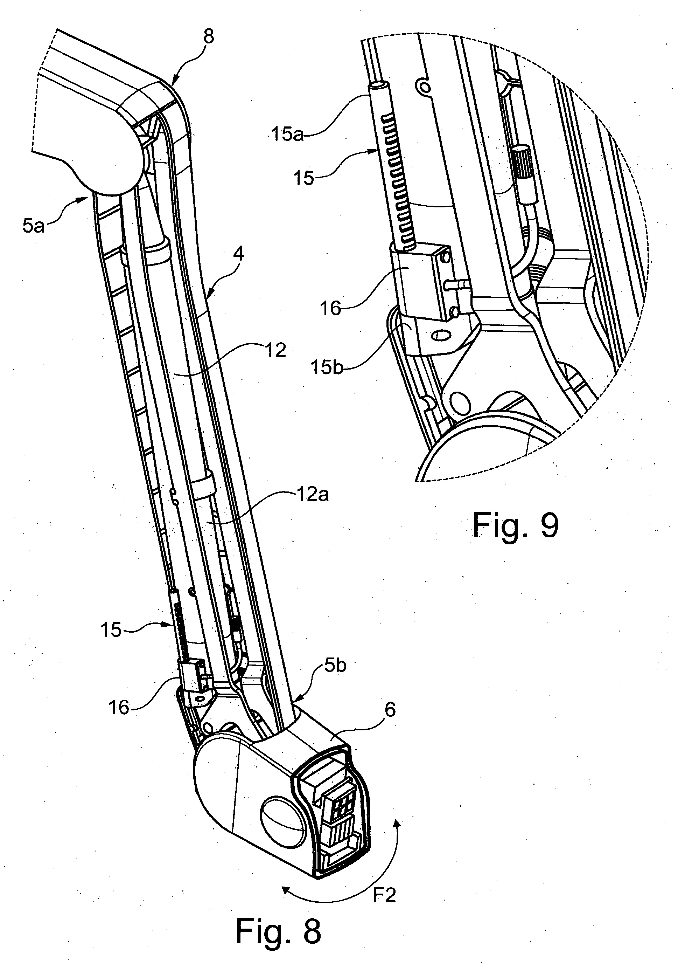

A titre d'exemple, le bras porteur 4 est représenté figure 5 dans une première position, légèrement abaissée et proche de l'horizontale, ce qui correspond à une position du loquet 15 près de l'extrémité libre 15a de la crémaillère 15 comme mieux visible sur les figures 6 et 7. On a représenté sur les figures 8 et 9 le bras porteur 4 dans une deuxième position, très abaissée et proche de la verticale, correspondant à une position du loquet 15 en butée contre l'extrémité 15b de la crémaillère 15 opposée à l'extrémité libre 15a.

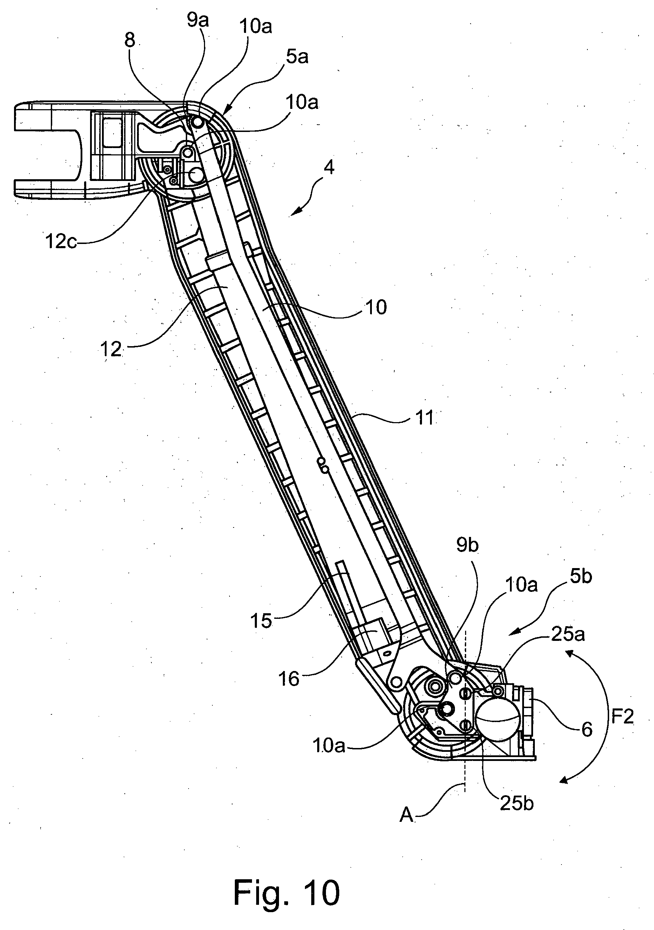

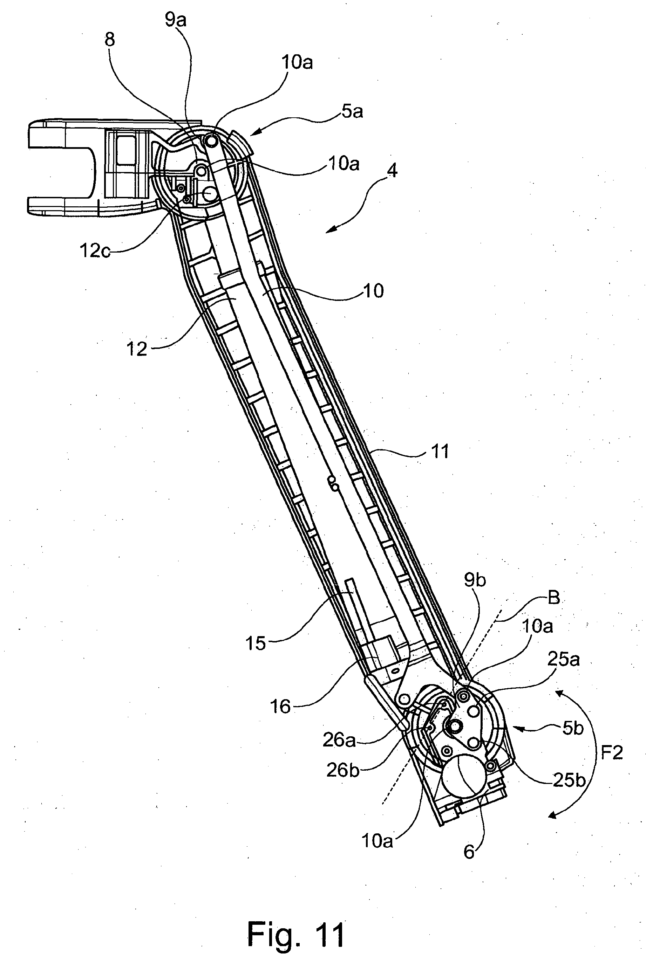

Les figures 10 et 11 montrent le bras porteur 4 sans capot avant pour mieux faire apparaître la fixation de la pièce de connexion 6 à l'extrémité libre 5 du bras porteur 4 selon deux configurations du bras porteur 4.

Plus précisément, dans une première configuration du bras porteur 4 représenté sur la figure 10, le bras porteur 4 est agencé pour que la pièce de connexion 6 disposée à l'extrémité libre 5 du bras porteur 4 conserve son orientation par rapport à la biellette fixe 9a lorsque le bras porteur 4 pivote autour de l'axe de pivot 8 selon la double flèche F2. Sur l'exemple représenté figure 10, la pièce de connexion 6 est fixée sur la biellette mobile 9b au niveau d'un premier couple de points 25a, 25b, ici au moyen de deux vis. De cette manière, la pièce de connexion 6 a un axe A d'orientation fixe par rapport à la biellette fixe 9a, qui reste parallèle à la biellette fixe 9a du parallélogramme lorsque le bras porteur 4 pivote autour de l'axe de pivot 8 selon la flèche F2 et change d'inclinaison. On comprend donc que, dans la configuration représentée sur la figure 10, l'axe A de la pièce de connexion 6 reste vertical lorsque le bras porteur 4 change d'inclinaison.

En variante, dans une deuxième configuration du bras porteur 4 représentée sur la figure 11, le bras porteur 4 est agencé pour que la pièce de connexion 6 s'oriente en suivant l'inclinaison du bras porteur 4 lorsque le bras porteur 4 pivote autour de l'axe de pivot 8 selon la double flèche F2. Ainsi, l'orientation de la pièce de connexion 6 est fixe par rapport à la bielle 10 mais variable par rapport à la biellette fixe 9a. Dans la figure 11, la pièce de connexion 6 est fixée sur la coque 11 au niveau d'un deuxième couple de points 26a, 26b, ici aussi au moyen de deux vis. On comprend que dans cette configuration, le premier couple de points 25a, 25b n'est pas utilisé. De cette manière, la pièce de connexion 6 a un axe B qui est solidaire de la coque 11 du bras porteur 4 et a donc une orientation variable lorsque le bras porteur 4 pivote autour de l'axe de pivot 8 selon la flèche F2 et change d'inclinaison selon la flèche F2. On comprend donc que, dans la configuration représentée sur la figure 11, l'axe B de la pièce de connexion 6 ne reste pas vertical lorsque le bras porteur 4 change d'inclinaison.

Pour procéder à l'installation d'une charge sur un bras porteur 4 selon l'invention, l'utilisateur commence par bloquer le bras porteur 4 dans une position de blocage souhaitée, d'inclinaison quelconque, en engageant les crans 17 du loquet 16 dans les encoches 21 de la crémaillère 15 prévues à cet effet. Pour ce faire, il peut avantageusement tourner sur la molette 24 du raccord flexible 23 lié aux crans 17. Puis, le cas échéant, l'utilisateur peut retirer la charge déjà en place sur le bras porteur 4.

L'utilisateur installe alors une charge sur la pièce de connexion 6.

En fonction le poids de la charge installée, l'utilisateur règle avantageusement la pression à l'intérieur du vérin 12 via la valve 13 en connectant un dispositif de réglage de la pression de façon connue en soi. La pression maximale à l'intérieur du vérin 12 est de préférence choisie en fonction de la charge de poids maximal susceptible d'être fixée en extrémité 5 du bras porteur 4. A titre d'exemple, la pression peut être réglée à 30 bars si le poids maximal de charge admissible est de 40 kilogrammes. L'utilisateur peut donc passer très facilement d'une charge à l'autre, plus lourde ou plus légère.

Une fois la pression à l'intérieur du vérin 12 ajustée, l'utilisateur peut débloquer le bras porteur 4, en désengageant les crans 17 du loquet 16 des encoches 21 de la crémaillère 15, en tournant la molette 24 du raccord flexible 23. Grâce à la force de retenue exercée par le vérin 12, le bras porteur 4 reste alors en position. Le bras porteur 4 est alors prêt à être déplacé dans toutes les positions d'inclinaison souhaitées par l'utilisateur en le faisant pivoter autour de l'axe de pivot 8 selon la flèche F2 représentée par exemple sur la figure 1.

En particulier, le réglage de la pression à l'intérieur du vérin 12 étant ajusté à la charge de poids maximal portée par le bras porteur 4, on peut avantageusement installer sur le bras porteur 4 une charge de poids variable, comme par exemple une poche de perfusion qui est destinée à se vider au cours du temps ou tout autre dispositif médical de poids variable.

II va de soi que la présente invention ne saurait être limitée à la description qui précède des modes de réalisation susceptibles de subir quelques modifications sans pour autant sortir du cadre de l'invention.

## Revendications

1. Bras porteur (4) de charge à position réglable comprenant une ossature globalement en forme de parallélogramme déformable pourvue d'au moins une biellette fixe (9a), une biellette mobile (9b), une première et une seconde bielles mobiles (10, 11) et une extrémité libre (5) apte à supporter une charge, un vérin (12) dont le corps (12a) et le piston (12b) sont l'un couplé à la biellette fixe (9b), l'autre à au moins l'une des première, seconde bielles (10, 11), ledit vérin (12) étant apte à actionner lesdites première, et seconde bielles (10, 11) par rapport à ladite biellette fixe (9b) de sorte à déformer ledit parallélogramme déformable, ledit vérin (12) étant de type à pression réglable de telle sorte que la pression à l'intérieur dudit vérin (12) soit réglable en fonction du poids de ladite charge portée par ledit bras porteur (4), **caractérisé en ce qu'**il comporte, en ladite extrémité libre (5), une pièce de connexion (6) apte à porter ladite charge et à être solidaire :
- dans une première configuration, de ladite biellette mobile (9b) de sorte à présenter un axe (A) d'orientation fixe par rapport à ladite biellette fixe (9a) et variable par rapport auxdites première et seconde bielles (10, 11) lorsque ledit bras porteur (4) change de position,
- dans une seconde configuration, de l'une au moins desdites première et seconde bielles (10, 11) de sorte à présenter un axe (B) d'orientation variable par rapport à ladite biellette fixe (9a) et fixe par rapport auxdites première et seconde bielles (10, 11) lorsque ledit bras porteur (4) change de position.

2. Bras porteur (4) selon la revendication 1, comprenant en outre un dispositif de blocage (14) de la position angulaire de ladite première, seconde bielle (10, 11) par rapport à ladite biellette fixe (9b), ledit dispositif de blocage (14) comprenant une crémaillère (15) et un loquet (16) solidaires l'un du corps (12a) du vérin (12), l'autre du piston (12b) du vérin (12), ledit loquet (16) étant agencé pour être mobile le long de ladite crémaillère (15) et apte à coopérer avec ladite crémaillère (15) de sorte à bloquer la position angulaire de ladite première, seconde bielle (10, 11) par rapport à ladite biellette fixe (9b) selon une pluralité de positions de blocage.

3. Bras porteur (4) selon la revendication 2, dans lequel ledit loquet (16) comprend au moins un cran (17) destiné à engager une encoche (21) de ladite crémaillère (15) pour bloquer ledit bras porteur (7) en position.

4. Bras porteur (4) selon l'une quelconque des revendications précédentes, comprenant en outre une valve (13) couplée audit vérin et destinée à relier ledit vérin (12) à un dispositif de réglage de la pression du vérin (12).

5. Bras porteur selon l'une quelconque des revendications précédentes, comprenant au moins une coque (11) formant ladite seconde bielle enveloppant ladite première bielle (10).

6. Bras porteur selon l'une quelconque des revendications précédentes, dans lequel ladite première bielle (10) présente une forme de Y dont la base est montée pivotante sur ladite biellette fixe (9a), dont l'extrémité libre d'une branche est montée pivotante sur ladite biellette mobile (9b) et l'extrémité libre de l'autre branche est reliée à une extrémité dudit vérin (12), l'autre extrémité dudit vérin étant pivotante par rapport à ladite biellette fixe (9a).

7. Dispositif de suspension (1) de charge comprenant au moins un bras articulé (2) ayant un premier bras (3) agencé pour être mobile en rotation selon un mouvement de rotation (F1) dans un premier plan et un bras porteur (4) selon l'une quelconque des revendications précédentes relié au premier bras (3) par une articulation pivot (8) agencée pour autoriser sa rotation selon un mouvement de rotation (F2) dans un deuxième plan distinct dudit premier plan.

## Patentansprüche

1. Tragarm (4) für eine Last mit verstellbarer Position, umfassend eine Konstruktion im Wesentlichen mit der Form eines verformbaren Parallelogramms, das mit mindestens einem festen Lenker (9a), einem beweglichen Lenker (9b), einer ersten und einer zweiten beweglichen Stange (10, 11) und einem freien Ende (5), das geeignet ist, eine Last zu tragen, versehen ist, und einen Zylinder (12), von dessen Gehäuse (12a) und dessen Kolben (12b) das bzw. der eine mit dem festen Lenker (9a) gekoppelt ist und das bzw. der andere zumindest mit einer der ersten und zweiten Stangen (10, 11), wobei der Zylinder (12) geeignet ist, die erste und die zweite Stange (10, 11) bezüglich des festen Lenkers (9a) derart zu betätigen, dass das verformbare Parallelogramm verformt wird, wobei der Zylinder (12) vom Typ mit einstellbarem Druck ist, derart, dass der Druck im Inneren des Zylinders (12) in Abhängigkeit vom Gewicht der von dem Tragarm (4) getragenen Last einstellbar ist, **dadurch gekennzeichnet, dass** er an dem freien Ende (5) ein Verbindungsstück (6) aufweist, das geeignet ist, die Last zu tragen und fest verbunden zu sein:
- in einer ersten Konfiguration mit dem beweglichen Lenker (9b), derart, dass es eine Achse (A) mit bezüglich des festen Lenkers (9a) fester und bezüglich der ersten und zweiten Stange (10, 11) variabler Ausrichtung aufweist, wenn der Tragarm (4) seine Position ändert,
- in einer zweiten Konfiguration mit zumindest einer der ersten und zweiten Stangen (10, 11), derart, dass es eine Achse (B) mit bezüglich des festen Lenkers (9a) variabler und bezüglich der ersten und zweiten Stangen (10, 11) fester Ausrichtung aufweist, wenn der Tragarm (4) seine Position ändert.

2. Tragarm (4) nach Anspruch 1, welcher außerdem eine Vorrichtung zur Arretierung (14) der Winkelposition der ersten und zweiten Stange (10, 11) bezüglich des festen Lenkers (9a) umfasst, wobei die Vorrichtung zur Arretierung (14) eine Zahnstange (15) und eine Klinke (16) umfasst, von denen die eine mit dem Gehäuse (12a) des Zylinders (12) und die andere mit dem Kolben (12b) des Zylinders (12) fest verbunden ist, wobei die Klinke (16) dafür ausgelegt ist, entlang der Zahnstange (15) beweglich zu sein, und geeignet ist, mit der Zahnstange (15) derart zusammenzuwirken, dass die Winkelposition der ersten und zweiten Stange (10, 11) bezüglich des festen Lenkers (9a) in mehreren Arretierpositionen arretiert wird.

3. Tragarm (4) nach Anspruch 2, wobei die Klinke (16) mindestens ein Rastelement (17) umfasst, das dazu bestimmt ist, in eine Kerbe (21) der Zahnstange (15) einzurasten, um den Tragarm (7) in seiner Position zu arretieren.

4. Tragarm (4) nach einem der vorhergehenden Ansprüche, welcher außerdem ein Ventil (13) umfasst, das mit dem Zylinder gekoppelt ist und dazu bestimmt ist, den Zylinder (12) mit einer Vorrichtung zur Einstellung des Drucks des Zylinders (12) zu verbinden.

5. Tragarm nach einem der vorhergehenden Ansprüche, welcher mindestens eine die zweite Stange bildende Schale (11) umfasst, welche die erste Stange (10) umhüllt.

6. Tragarm nach einem der vorhergehenden Ansprüche, wobei die erste Stange (10) die Form eines Y aufweist, dessen Fuß schwenkbar an dem festen Lenker (9a) angebracht ist, dessen freies Ende eines Schenkels schwenkbar an dem beweglichen Lenker (9b) angebracht ist und dessen freies Ende des anderen Schenkels mit einem Ende des Zylinders (12) verbunden ist, wobei das andere Ende des Zylinders bezüglich des festen Lenkers (9a) schwenkbar ist.

7. Aufhängevorrichtung (1) für eine Last, welche mindestens einen Gelenkarm (2) umfasst, der einen ersten Arm (3) aufweist, der dafür ausgelegt ist, drehbeweglich gemäß einer Drehbewegung (F1) in einer ersten Ebene zu sein, und einen Tragarm (4) nach einem der vorhergehenden Ansprüche, der mit dem ersten Arm (3) durch ein Drehgelenk (8) verbunden ist, das dafür ausgelegt ist, seine Drehung gemäß einer Drehbewegung (F2) in einer zweiten Ebene, die von der ersten Ebene verschieden ist, zu ermöglichen.

## Claims

1. Adjustable-position load supporting arm (4) comprising a framework having on the whole the shape of a deformable parallelogram provided with at least a fixed link (9a), a mobile link (9b), a first and a second mobile connecting rod (10, 11) and a free end (5) able to support a load, a jack (12) comprising a body (12a) and a piston (12b) one of which is coupled with the fixed link (9a), the other of which is coupled with at least one of the first and second connecting rods (10, 11), said jack (12) being able to actuate said first and second connecting rods (10, 11) with respect to said fixed link (9a) so as to deform said deformable parallelogram, said jack (12) being of the adjustable-pressure type so that the pressure inside said jack (12) is adjustable according to the weight of said load supported by said supporting arm (4), **characterized in that** it comprises, at said free end (5), a connecting piece (6) able to support said load and to be firmly connected to:
- in a first configuration, said mobile link (9b) so as to have an axis of orientation (A) that is fixed with respect to said fixed link (9a) and variable with respect to said first and second connecting rods (10, 11) when said supporting arm (4) shifts position,
- in a second configuration, at least one of said first and second connecting rods (10, 11) so as to have an axis of orientation (B) that is variable with respect to said fixed link (9a) and fixed with respect to said first and second connecting rods (10, 11) when said supporting arm (4) shifts position.

2. Supporting arm (4) according to claim 1, moreover comprising a device (14) for blocking the angular position of said first, second connecting rod (10, 11) with respect to said fixed link (9a), said blocking device (14) comprising a toothed rack (15) and a latch (16) one of which is firmly connected to the body (12a) of the jack (12), the other of which is firmly connected to the piston (12b) of the jack (12), said latch (16) being arranged so as to be mobile along said toothed rack (15) and able to cooperate with said toothed rack (15) so as to block the angular position of said first, second connecting rod (10, 11) with respect to said fixed link (9a) in a plurality of blocking positions.

3. Supporting arm (4) according to claim 2, in which said latch (16) comprises at least one cog (17) intended to be inserted into a notch (21) in said toothed rack (15) in order to block said supporting arm (7) in position.

4. Supporting arm (4) according to anyone of the preceding claims, moreover comprising a valve (13) coupled with said jack and intended to connect said jack (12) to a device for adjusting the pressure in the jack (12).

5. Supporting arm according to anyone of the preceding claims, comprising at least one shell (11) forming said second connecting rod jacketing said first connecting rod (10).

6. Supporting arm according to anyone of the preceding claims, in which said first connecting rod (10) has the shape of a Y whose basis is pivotally mounted on said fixed link (9a), one branch of which has a free end pivotally mounted on said mobile link (9b) and the other branch of which has its free end connected to one end of said jack (12), the other end of said jack being able to pivot with respect to said fixed link (9a).

7. Load suspending device (1) comprising at least one articulated arm (2) having a first arm (3) arranged so as to be mobile rotatable to a rotational movement (F1) in a first plane and a supporting arm (4) according to anyone of the preceding claims connected to the first arm (3) by means a pivot joint (8) arranged so as to rotate according to a rotational movement (F2) in a second plane distinct from said first plane.
